# EUROPEAN PATENT SPECIFICATION

(11) **EP 4 397 658 B1**
(45) Date of publication and mention of the grant of the patent: **22.10.2025**
(21) Application number: 23740545.1
(22) Date of filing: 17.01.2023
(51) Int. Cl.: C07D 251/30, C08G 73/06, C07D 251/34, C08F 26/00

(54) **COMPOUND, METHOD FOR PREPARING SAME, AND SINGLE MOLECULE, OLIGOMER AND POLYMER DERIVED FROM SAME**
VERBINDUNG, VERFAHREN ZUR HERSTELLUNG DAVON UND EINZELMOLEKÜL, OLIGOMER UND POLYMER DARAUS
COMPOSÉ, SON PROCÉDÉ DE PRÉPARATION, MOLÉCULE UNIQUE, OLIGOMÈRE ET POLYMÈRE DÉRIVÉS DE CELUI-CI

(30) Priority: 17.01.2022 KR 20220006320
(43) Date of publication of application: 10.07.2024
(73) Proprietor: LG Chem, Ltd., Yeongdeungpo-gu Seoul 07336 (KR)
(72) Inventor: JUNG, Jihye, Daejeon 34122 (KR); PARK, Beomsu, Daejeon 34122 (KR); KIM, Hyun Cheol, Daejeon 34122 (KR)
(74) Representative: Goddar, Heinz J.
(86) International application number: PCT/KR2023/000792
(87) International publication number: WO 2023/136702

(56) References cited:
- EP-A1- 0 937 717
- EP-A1- 3 498 756
- EP-A1- 3 780 226
- EP-A1- 4 008 741
- WO-A1-2021/024964
- JP-A- H05 117 247
- KR-A- 20200 110 424
- GRYAZNOVA T. V.; KHRIZANFOROV M. N.; KHOLIN K. V.; VOROTYNTSEV M. A.; GOR’KOV K. V.; TALAGAEVA N. V.; DMITRIEVA M. V.; ZOLOT: "Palladium Nanoparticles–Polypyrrole Composite as Effective Catalyst for Fluoroalkylation of Alkenes", CATALYSIS LETTERS, J.C. BALTZER, NEW YORK, vol. 148, no. 10, 18 August 2018 (2018-08-18), New York , pages 3119 - 3125, XP036589534, ISSN: 1011-372X, DOI: 10.1007/s10562-018-2524-z
- ZANG XIONG, WU LIN, CAI LU, LI ZHANXIONG: "Synthesis of Perfluoroalkyl Siloxane with C=C and C-C Bridged Groups Through Single Electron Transfer Reaction", PHOSPHORUS, SULFUR, AND SILICON AND THE RELATED ELEMENTS, vol. 190, no. 7, 3 July 2015 (2015-07-03), pages 1035 - 1044, XP093078695, ISSN: 1042-6507, DOI: 10.1080/10426507.2014.965814
- YAJIMA TOMOKO, JAHAN ISHRAT, TONOI TAKAYUKI, SHINMEN MANAMI, NISHIKAWA AYA, YAMAGUCHI KANAKO, SEKINE IZUMI, NAGANO HAJIME: "Photoinduced addition and addition–elimination reactions of perfluoroalkyl iodides to electron-deficient olefins", TETRAHEDRON, ELSEVIER SIENCE PUBLISHERS, AMSTERDAM, NL, vol. 68, no. 34, 1 August 2012 (2012-08-01), AMSTERDAM, NL , pages 6856 - 6861, XP055884416, ISSN: 0040-4020, DOI: 10.1016/j.tet.2012.06.028

## Description

### [Technical Field]

The present specification relates to a compound, a method for preparing the same, and an oligomer and a polymer derived from the compound.

### [Background Art]

Triallyl isocyanurate is useful as a cross-linking agent having excellent heat resistance and chemical resistance, and is expected to be used in a wide range of fields such as electronic materials, liquid crystals, semiconductors, and solar cells. For example, in printed circuit boards, that is, plate-like or film-like parts that constitute electronic circuits by fixing a large number of electronic parts such as integrated circuits, resistors, and condensers to a surface thereof and connecting the parts through wiring, triallyl isocyanurate is used as a sealing material for preventing penetration of materials such as liquids or gases into the parts.

However, triallyl isocyanurate has high relative permittivity and high dissipation factor, so that research has been continuously conducted to reduce them.
EP 3 780 226 A1 discloses a compound:

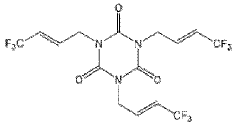

### [Detailed Description of the Invention]

### [Technical Problem]

The present specification provides a compound, a method for preparing the same, and and an oligomer derived from the compound.

### [Technical Solution]

An exemplary embodiment of the present specification provides a compound of the following Chemical Formula 1.

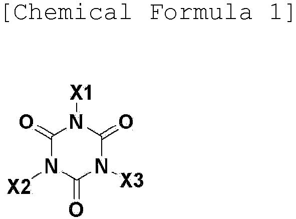

In Chemical Formula 1,
at least one of X1 to X3 is 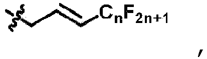 and the others are an allyl group,
n is an integer from 4 to 12, and 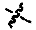 is a moiety bonded to Chemical Formula 1.

An exemplary embodiment of the present specification provides a method for preparing a compound of the following Chemical Formula 1, the method including: (s1) putting triallyl isocyanurate, CₙF₂ₙ₊₁X4, a first base, and a first solvent into a container and stirring the resulting mixture under nitrogen gas; (s2) adding a radical initiator thereto and stirring the resulting mixture; and (s3) adding a second solvent and a second base thereto.

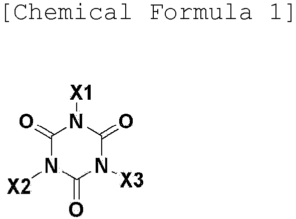

In Chemical Formula 1,
at least one of X1 to X3 is 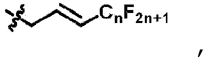 and the others are an allyl group,
n is an integer from 4 to 12, 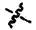 is a moiety bonded to Chemical Formula 1, and
X4 is a halogen group.

An exemplary embodiment of the present specification provides a mixture including two or more of the compounds.

Further, still another exemplary embodiment of the present specification provides an oligomer including a monomer derived from the compound.

Yet another exemplary embodiment of the present specification provides a polymer including a monomer derived from the compound.

Further embodiments are disclosed in the dependent claims.

### [Advantageous Effects]

The compound according to an exemplary embodiment of the present specification, and the oligomer and the polymer derived therefrom include a good release group and a structure capable of being cross-linked, so that various derivatives can be prepared.

The compound according to an exemplary embodiment of the present specification can be used as a polyfunctional monomer to exhibit the effects of achieving low refractive index, low relative permittivity, low surface energy and low dissipation factor.

In the method for preparing a compound according to an exemplary embodiment of the present specification, the reaction is performed under mild conditions with minimal side reactions, so that the yield of the compound of Chemical Formula 1, which is a target compound, is increased.

### [Brief Description of Drawings]

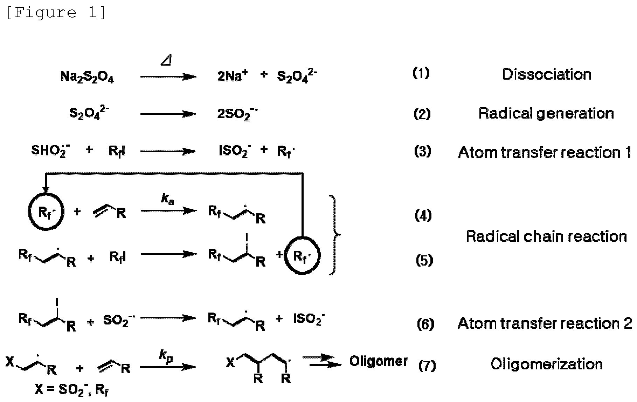

FIG. 1 is a view describing the mechanism of the method for preparing the compound of Chemical Formula 1.

### [Best Mode]

Hereinafter, the present specification will be described in more detail.

An exemplary embodiment of the present specification provides a compound of the following Chemical Formula 1.

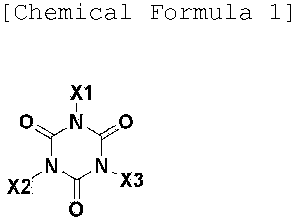

In Chemical Formula 1,
at least one of X1 to X3 is 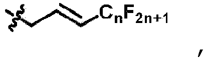 and the others are an allyl group,
n is an integer from 4 to 12, and 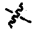 is a moiety bonded to Chemical Formula 1.

The compound of Chemical Formula 1 according to an exemplary embodiment of the present specification includes a perfluoroalkyl group including fluorine having the effects of achieving low refractive index, low relative permittivity, and low dissipation factor, and a double bond that reduces the degree of freedom, and is used as a polyfunctional monomer as a triallyl isocyanurate derivative that serves as a cross-linking agent, so that it is possible to exhibit the effects of achieving low refractive index, low relative permittivity, low surface energy and low dissipation factor.

According to an exemplary embodiment of the present specification, any one of X1 to X3 is 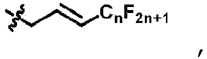 and the others are an allyl group.

According to an exemplary embodiment of the present specification, any two of X1 to X3 are 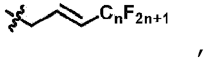 and the other is an allyl group.

According to an exemplary embodiment of the present specification, X1 to X3 are each

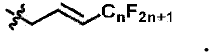

According to an exemplary embodiment of the present specification, n is 4, 6 or 8.

According to an exemplary embodiment of the present specification, the 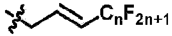 is any one of the following structures.

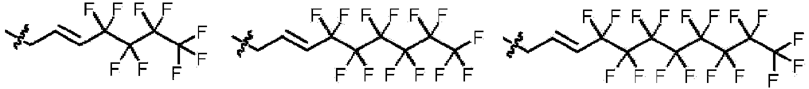

According to an exemplary embodiment of the present specification, when n of the 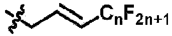 is within the above range, compounds with various molecular weights may be prepared because the chain length of a perfluoroalkyl group is easily adjusted. In addition, when a perfluoroalkyl group is included, it is possible to exhibit effects of achieving low refractive index, low relative permittivity, low surface energy and low dissipation factor.

According to an exemplary embodiment of the present specification, Chemical Formula 1 is any one selected from the following compounds.

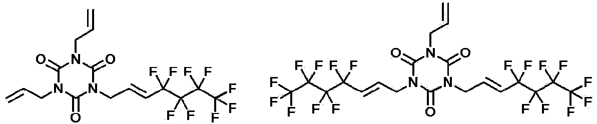

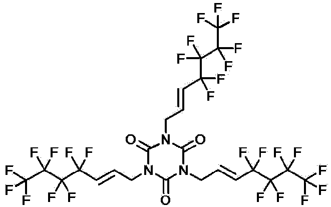

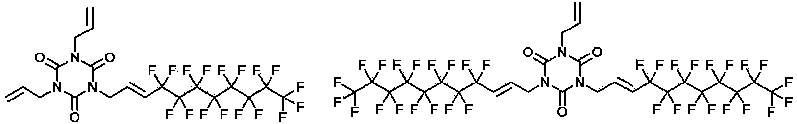

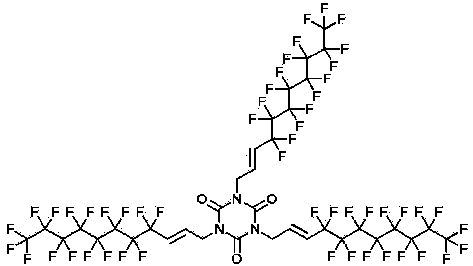

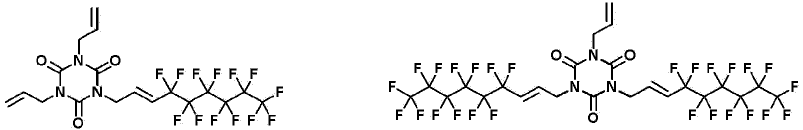

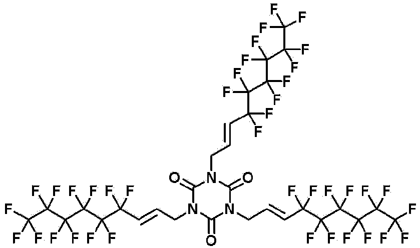

An exemplary embodiment of the present specification provides a method for preparing a compound of the following Chemical Formula 1, the method including: (s1) putting triallyl isocyanurate, CₙF₂ₙ₊₁X4, a first base, and a first solvent into a container and stirring the resulting mixture under nitrogen gas; (s2) adding a radical initiator thereto and stirring the resulting mixture; and (s3) adding a second solvent and a second base thereto.

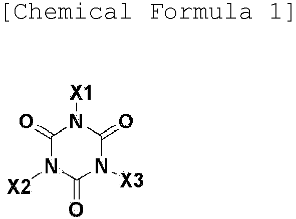

In Chemical Formula 1,
at least one of X1 to X3 is 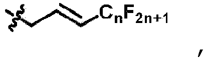 and the others are an allyl group,
n is an integer from 4 to 12, 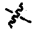 is a moiety bonded to Chemical Formula 1, and
X4 is a halogen group.

According to an exemplary embodiment of the present specification, X4 is an iodine group (-I).

According to an exemplary embodiment of the present specification, the reaction temperature in Step (s1) is 0°C to room temperature. When the above reaction is performed within the above reaction temperature range, the reaction conditions are not tricky but mild, and side reactions that appear as the temperature rises are suppressed, so that the yield of the compound of Chemical Formula 1, which is a final target, is increased. In addition, various temperature ranges may be set according to the range of n and the molecular weight of the compound of Chemical Formula 1 in Step (s1).

In the present specification, room temperature means 20±5°C under atmospheric pressure.

The first base according to an exemplary embodiment of the present specification is a non-nucleophilic base.

The first base according to an exemplary embodiment of the present specification may be potassium carbonate (K₂CO₃), sodium hydrogen carbonate (NaHCO₃), potassium hydrogen carbonate (KHCO₃), 1,8-diazabicyclo[5,4,0]undec-7-ene (DBU) or any mixture thereof, but is not limited thereto, and a salt used in the related art may be used.

According to an exemplary embodiment of the present specification, the first solvent is an organic solvent.

According to an exemplary embodiment of the present specification, the first solvent is selected among hexane, heptane, toluene, benzene, acetonitrile, dichloromethane (methylene chloride), dichloroethane, trichloroethane, chloroform, dichloroform, nitromethane, dibromomethane, cyclopentanone, cyclohexanone, fluorobenzene, bromobenzene, chlorobenzene, xylene, mesitylene, ethylacetate or any mixture thereof, but is not limited thereto, and an organic solvent used in the related art may be used.

According to an exemplary embodiment of the present specification, the radical initiator is a water-soluble initiator.

According to an exemplary embodiment of the present specification, the radical initiator is any one of potassium persulfate (KPS), ammonium persulfate (APS), potassium hydrosulfite, sodium hydrosulfite, sodium persulfate, or hydrogen peroxide.

According to an exemplary embodiment of the present specification, the reaction temperature in Step (s2) is 0°C or higher. Specifically, the reaction temperature in Step (s2) is 0°C.

When Step (s2) is performed at the above reaction temperature, side reactions are suppressed, so that the yield of the compound of Chemical Formula 1, which is a final target, is increased.

According to an exemplary embodiment of the present specification, the second solvent is an organic solvent.

According to an exemplary embodiment of the present specification, the second solvent is selected among hexane, heptane, toluene, benzene, acetonitrile, dichloromethane (methylene chloride), dichloroethane, trichloroethane, chloroform, dichloroform, nitromethane, dibromomethane, cyclopentanone, cyclohexanone, fluorobenzene, bromobenzene, chlorobenzene, xylene, mesitylene, ethylacetate or any mixture thereof, but is not limited thereto, and an organic solvent used in the related art may be used.

According to an exemplary embodiment of the present specification, the second base is a non-nucleophilic base.

The second base according to an exemplary embodiment of the present specification may be potassium carbonate (K₂CO₃), sodium hydrogen carbonate (NaHCO₃), potassium hydrogen carbonate (KHCO₃), 1,8-diazabicyclo[5,4,0]undec-7-ene (DBU) or any mixture thereof, but is not limited thereto, and a salt used in the related art may be used.

According to an exemplary embodiment of the present specification, the adding of the second base in Step (s3) may be performed by dropwise addition, dropwise dropping, or a method used in the related art.

According to an exemplary embodiment of the present specification, the reaction temperature in Step (s3) is 0°C to room temperature. When the above reaction is performed within the above reaction temperature range, the reaction conditions are not tricky but mild, and side reactions that appear as the temperature rises are suppressed, so that the yield of the compound of Chemical Formula 1, which is a final target, is increased. In addition, various temperature ranges may be set according to the range of n and the molecular weight of the compound of Chemical Formula 1 in Step (s3).

The mechanism of the preparation method according to an exemplary embodiment of the present specification is described in the following FIG. 1. (1) The radical initiator is dissociated under basic conditions, (2) radicals are generated, and (3) the first atom transfer reaction occurs. The hydrogen of the initiator is dissociated in (3) and bonded to a halogen group (an iodine group) bonded to perfluoroalkyl, and the perfluoroalkyl becomes a radical. Thereafter, by the radical chain reaction of (4) and (5), (4) the radicalized perfluoroalkyl group and the allyl group of the triallyl isocyanurate are bonded to form a radical, (5) the halogen group (the iodine group) bonded to the perfluoroalkyl is bonded to the radical to produce an intermediate compound in which the halogen group (the iodine group) is bonded, and (6) the halogen group is dissociated in the intermediate compound by the second atom transfer reaction, and a removal reaction occurs to produce a compound of Chemical Formula 1 including a perfluoroalkyl group including a double bond. Additionally, (7) oligomerization may occur due to the radicals generated by the reaction of (6).

In FIG. 1, *Kₐ* is the addition rate constant of radical to substrate (R_{f}), and *Kₚ* is the oligomerization rate constant of substrate.

According to an exemplary embodiment of the present specification, a step of obtaining an intermediate compound after Step (s2) and a step of obtaining the compound of Chemical Formula 1 after Step (s3) are further included.

The obtaining step of the above according to an exemplary embodiment of the present specification includes:
(s41) a quench and layer separation step;
(s42) a drying step;
(s43) a filtration step;
(s44) a concentration step; and
(s45) an obtaining step.

According to an exemplary embodiment of the present specification, Step (s41) (quench and layer separation step) is a step of terminating the reaction by adding a reaction termination material, and separating the layers.

According to an exemplary embodiment of the present specification, as the reaction termination material of Step (s41), distilled water, an aqueous ammonium chloride solution, an aqueous sodium hydrogen carbonate solution, an aqueous KF (saturated) solution, an aqueous HCl solution, an aqueous NaCl (saturated) solution, chloroform, dichloromethane, ethyl acetate, and the like may be used, but the reaction termination material is not limited thereto. Preferably, as the reaction termination material, an aqueous ammonium chloride solution, an aqueous sodium hydrogen carbonate solution, an aqueous NaCl solution, and ethyl acetate are used.

In Step (s41), the order in which the reaction termination and layer separation are performed is not limited to a specific order. As an exemplary embodiment, reaction termination and layer separation may be simultaneously performed, and as another exemplary embodiment, the reaction is terminated by adding a reaction termination material, and then layers may be separated. However, the progression order is not limited to the above-described example.

Since the radical initiator remaining after Step (s2) is water-soluble, the radical initiator may be dissolved in water and removed in Step (s41).

According to an exemplary embodiment of the present specification, Step (s42) (drying step) is performed by adding a desiccant to any one layer of the material layer-separated in Step (s41). For example, when chloroform exemplified above is used as the reaction termination material, Step (s42) is performed by adding a desiccant to a chloroform layer which is a lower layer, that is, an organic layer.

Examples of the desiccant include magnesium sulfate, sodium sulfate, and the like, but are not limited thereto. According to a preferred exemplary embodiment, the desiccant is magnesium sulfate.

In the present specification, the desiccant is added in a sufficient amount required for typical drying in the art.

According to an exemplary embodiment of the present specification, Step (s43) (filtration step) may be performed by a method known in the art. As a representative example, it is possible to use a method of inhaling air using an inhaler and filtering impurities including a desiccant, and the like, which are separated from a target product with a filter, or a method of removing other impurities using a solvent that does not dissolve a target product, but dissolves other impurities by utilizing the difference in solubility in a specific solvent.

According to an exemplary embodiment of the present specification, Step (s44) (concentration step) may be performed by a method known in the art. The step may be performed by evaporating the solvent using a vacuum rotary evaporator as a representative example, but the representative example is not limited thereto.

According to an exemplary embodiment of the present specification, additional filtration and drying steps may be added after Step (s44).

It is possible to include a drying step before the obtaining step (s45), and the drying step is a step of drying the intermediate compound, or the compound of Chemical Formula 1. The drying step is different from Step (s42) and may be performed by a method known in the art, representative examples thereof include vacuum oven drying, spray drying, flash drying, and the like, and preferably, the compound of Chemical Formula 1 may be dried by vacuum oven drying.

According to an exemplary embodiment of the present specification, Step (s45) (obtaining step) is a step of obtaining the intermediate compound, or the compound of Chemical Formula 1, which is concentrated (dried) in Step (s44).

According to an exemplary embodiment of the present specification, in the obtaining step, Steps (s42) to (s43) may be performed three or more times, if necessary.

According to an exemplary embodiment of the present specification, provided is a mixture including two or more of the compounds.

According to another exemplary embodiment of the present specification, two or more compounds included in the mixture are the same as or different from each other. That is, it means that the two or more compounds are the same as or different from each other while having the structure of Chemical Formula 1.

According to still another exemplary embodiment of the present specification, the mixture may further include a compound different from the compound of Chemical Formula 1.

According to an exemplary embodiment of the present specification, provided is an oligomer including a monomer derived from the compound.

Those skilled in the art may understand the term "monomer" described in the present specification as a state in which a compound is polymerized to be linked to the main chain of the oligomer.

In the present specification, for example, the "monomer derived from the compound of Chemical Formula 1" is a repeating unit that constitutes the main chain in the polymer, and means that an allyl group of the compound of Chemical Formula 1 may form a radical to become a monomer, and a halogen group may be used as an electron donor to introduce a monomer or end group which makes up the main chain of other oligomers.

Furthermore, radicals generated by the release of the halogen group used as the electron donor may also react with other allyl group-containing oligomers or the above monomers.

The compound according to an exemplary embodiment of the present specification, and an oligomer derived therefrom include a good release group and a structure capable of being cross-linked, so that various derivatives can be prepared.

According to an exemplary embodiment of the present specification, provided is a polymer including a monomer derived from the compound.

Those skilled in the art will understand the term "monomer" described in the present specification as a state in which a compound is polymerized to be linked to the main chain of the polymer.

In the present specification, for example, the "monomer derived from the compound of Chemical Formula 1" is a repeating unit that constitutes the main chain in the polymer, and means that a vinyl group of the compound of Chemical Formula 1 may form a radical to become a monomer, and a monomer or end group which makes up the main chain of other polymers may be introduced.

According to an exemplary embodiment of the present specification, the polymer may further include an additional monomer, and the additional monomer is not limited.

According to an exemplary embodiment of the present specification, the polymer may be an alternating polymer or a random polymer, but is not limited thereto.

Further, in the present specification, even when a monomer included in the polymer is mentioned, the monomer is not limited to including only the mentioned monomer, and other monomers in addition to the aforementioned monomer may additionally be included as co-monomers within a range not departing from the object of the present invention.

A compound according to an exemplary embodiment of the present specification, and an oligomer and a polymer derived therefrom are used as an electronic material, an organic insulating material, and/or a substrate material, but are not limited thereto.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below in detail. The Examples of the present specification are provided to explain the present specification more completely to a person with ordinary skill in the art.

### Example 1.

After 9.9 g of triallyl isocyanurate, 0.101 g of NaHCO₃ and 20.7 mL of nonafluoro-1-iodobutane were dissolved in 100 mL of water and 50 mL of acetonitrile in a 250 mL two-neck round-bottom flask and N₂ bubbling was performed at room temperature for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 44 g of an intermediate compound which was derived from perfluorobutyl iodide.

After 14.38 g of the intermediate compound was dissolved in 13 mL of methylene chloride in a 100 mL two-neck round-bottom flask, 5.2 mL of 1,8-diazabicyclo[5,4,0]undec-7-ene (DBU) was added dropwise thereto over 1 minute. After 1 hour, 30 mL of 1N HCl was added, the resulting mixture was stirred for 30 minutes, then transferred to a separatory funnel and washed with 100 mL of HCl, 100 mL of a saturated NaHCO₃ solution and 200 mL of water, and then the organic layer was dried by adding 10 g of MgSO₄ thereto. Thereafter, filtration was performed and the solvent was evaporated to obtain 10 g of a final product. Among the final products measured by ¹H-NMR, the mass ratios of Compounds 1-1 and 1-2 and Oligomer 1 and the weight average molecular weights measured by gel permeation chromatography (GPC) are shown in the following Table 1.

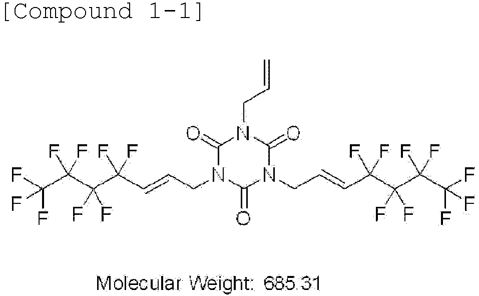

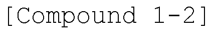

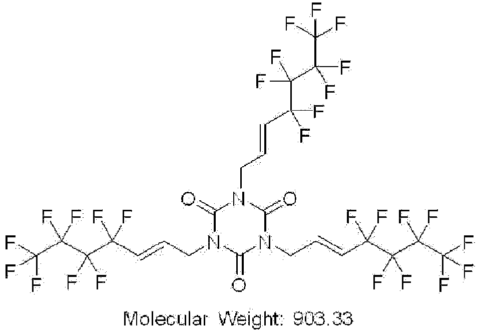

**[Table 1]**

| Structure | Compound 1-1 | Compound 1-2 | Oligomer 1 | Molecular weight (by ¹H-NMR) |
|---|---|---|---|---|
| Mass ratio (weight %) | 87 | 8 | 5 | 1300 |

Oligomer 1 of Example 1 is derived from any one or more of Compounds 1-1 and 1-2, and means a product whose molecular weight exceeds that of Compound 1-2, whose molecular weight is the maximum unit as a single molecule.

### Example 2.

After 9.97g of triallyl isocyanurate, 0.101 g of NaHCO₃ and 31.7 mL of heptadecafluoro-n-octyl iodide were dissolved in 100 mL of water and 40 mL of acetonitrile in a 250 mL two-neck round-bottom flask and N₂ bubbling was performed at 0°C for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 53 g of an intermediate product which was derived from perfluorooctyl iodide.

After 13.20 g of the intermediate compound was dissolved in 10 mL of methylene chloride in a 100 mL two-neck round-bottom flask, 3.2 mL of 1,8-diazabicyclo[5,4,0]undec-7-ene (DBU) was added dropwise thereto over 1 minute. After 1 hour, 30 mL of 1N HCl was added, the resulting mixture was stirred for 30 minutes, then transferred to a separatory funnel and washed with 100 mL of HCl, 100 mL of a saturated NaHCO₃ solution and 200 mL of water, and then the organic layer was dried by adding 10 g of MgSO₄ thereto. Thereafter, filtration was performed and the solvent was evaporated to obtain 10 g of a final product. Among the final products measured by ¹H-NMR, the mass ratios of Compounds 2-1 and 2-2 and Oligomer 2 and the weight average molecular weights measured by gel permeation chromatography (GPC) are shown in the following Table 2.

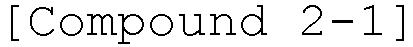

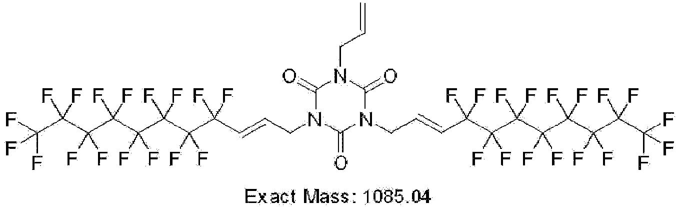

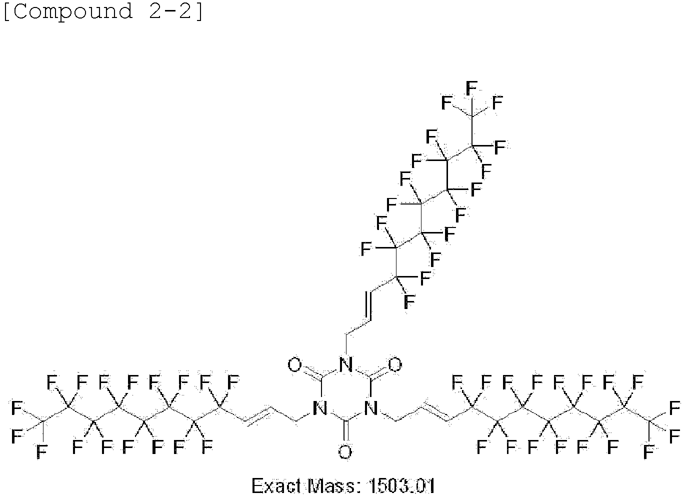

**[Table 2]**

| Structure | Compound 2-1 | Compound 2-2 | Oligomer 2 | Molecular weight (by ¹H-NMR) |
|---|---|---|---|---|
| Mass ratio (weight %) | 13 | 86 | 1 | 1100 |

Oligomer 2 of Example 2 is derived from any one or more of Compounds 2-1 and 2-2, and means a product whose molecular weight exceeds that of Compound 2-2, whose molecular weight is the maximum unit as a single molecule.

Through Tables 1 and 2, the compound of Chemical Formula 1 includes a perfluoroalkyl group including fluorine having the effects of achieving low relative permittivity and low dissipation factor, and a double bond that reduces the degree of freedom, and is used as a polyfunctional monomer as a triallyl isocyanurate derivative that serves as a cross-linking agent, so that it is possible to exhibit the effects of achieving low refractive index, low relative permittivity, low surface energy (low surface tension) and low dissipation factor, and thus the compound of Chemical Formula 1 can be used as an electronic material, an organic insulating material, and/or a substrate material.

### Evaluation Example: Measurement of refractive index and surface tension

The refractive indices of the final products prepared in Examples 1 and 2 and triallyl isocyanurate, which is the material of Comparative Example 1, were measured at 25°C using RX-5000α (ATAGO Co., Ltd.).

Further, the surface tensions of the final products prepared in Examples 1 and 2 and triallyl isocyanurate, which is the material of Comparative Example 1, were measured using Tensiometer K11 (KRUSS GmbH). The surface tension was measured by making a 0.5 wt% Propylene glycol monomethyl ether acetate (PGMEA) solution at 27.8°C to 28.1°C.

As the triallyl isocyanurate used in Comparative Example 1, a product manufactured by TCI Co., Ltd. was used.

**[Table 3]**

| Structure | Comparative Example 1 (Triallyl isocyanurate) | Evaluation Example 1 (Final product of Example 1) | Evaluation Example 2 (Final product of Example 2) |
|---|---|---|---|
| Refractive index | 1.51 | 1.40 | 1.36 |
| Surface tension (mN/m) | 25.6 | 25.0 | 21.2 |

In Table 3, it could be seen that the compound of the present specification has a lower refractive index and a lower surface tension than the triallyl isocyanurate of Comparative Example 1, which is a material in the related art. Therefore, the compound of the present specification may be used as an electronic material, an organic insulating material, and/or a substrate material.

### Evaluation Example: Measurement of dielectric characteristics

A coating composition was prepared by mixing the triallyl isocyanurate of Comparative Example 1 with OPE-2st (Mitsubishi Gas Chemical Company, Inc.) and a solvent such as toluene. A copper (Cu) foil was coated with the prepared coating composition and dried. After the dried film was vacuum-pressed at 225°C, dielectric characteristics were measured. The measured relative permittivity (Dk) value was 2.60.

A coating composition was prepared by mixing the final products of Examples 1 and 2 at a ratio of 1:1, instead of the triallyl isocyanurate of Comparative Example 1, and the dielectric characteristics were measured in the same manner. As a result, it was confirmed that the relative permittivity (Dk) value was lowered to 2.525.

Through the above results, the compound of Chemical Formula 1 may be used to lower the permittivity when the compound is prepared as a substrate material composition and applied to a copper clad laminate (CCL) or the like.

## Claims

1. A compound of the following Chemical Formula 1: 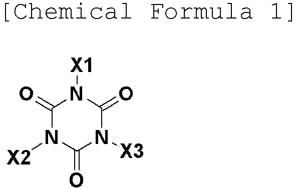 wherein, in Chemical Formula 1,
at least one of X1 to X3 is 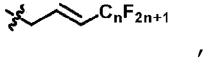 and the others are an allyl group,
n is an integer from 4 to 12, and 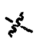 is a moiety bonded to Chemical Formula 1.

2. The compound of claim 1, wherein the 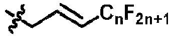 is any one of the following structures:

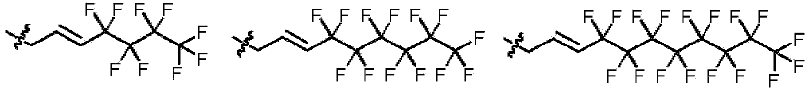

3. The compound of claim 1, wherein Chemical Formula 1 is any one selected from the following compounds:

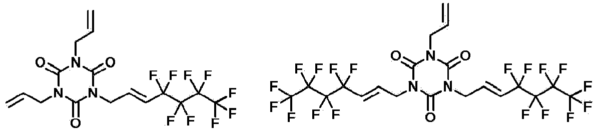

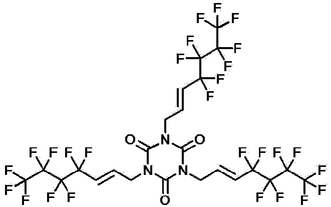

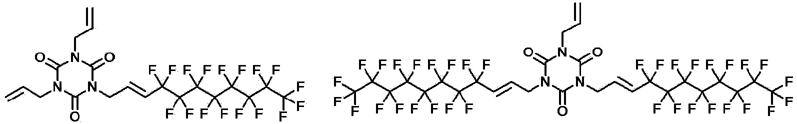

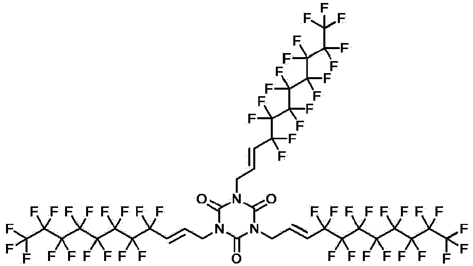

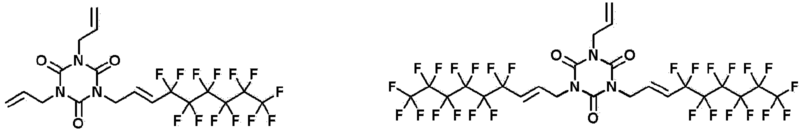

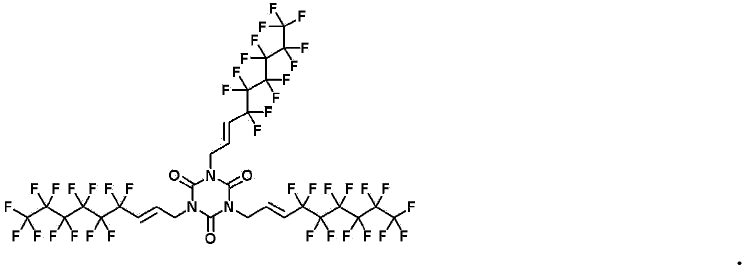

4. A method for preparing a compound of the following Chemical Formula 1, the method comprising:
(s1) putting triallyl isocyanurate, CₙF₂ₙ₊₁X4, a first base, and a first solvent into a container and stirring the resulting mixture under nitrogen gas;
(s2) adding a radical initiator thereto and stirring the resulting mixture; and
(s3) adding a second solvent and a second base thereto, 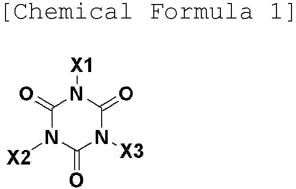
wherein, in Chemical Formula 1,
at least one of X1 to X3 is 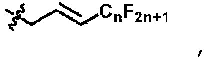 and the others are an allyl group,
n is an integer from 4 to 12, 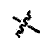 is a moiety bonded to Chemical Formula 1, and X4 is a halogen group.

5. A mixture comprising two or more of the compounds of any one of claims 1 to 3.

6. An oligomer comprising a monomer derived from the compound of any one of claims 1 to 3.

7. A polymer comprising a monomer derived from the compound of any one of claims 1 to 3.

8. The polymer of claim 7, wherein the polymer further comprises an additional monomer.

## Patentansprüche

1. Verbindung der folgenden chemischen Formel 1: 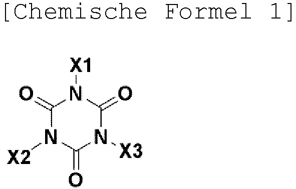 wobei in der chemischen Formel 1
mindestens eines von X1 bis X3 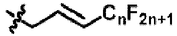 ist und die anderen eine Allylgruppe sind,
n eine ganze Zahl von 4 bis 12 ist und 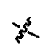 eine Einheit ist, die an die chemische Formel 1 gebunden ist.

2. Verbindung nach Anspruch 1, wobei das 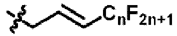 eine der folgenden Strukturen ist:

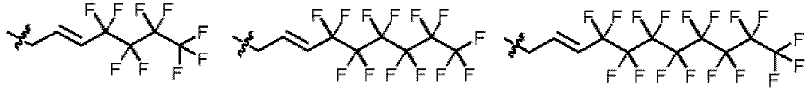

3. Verbindung nach Anspruch 1, wobei die chemische Formel 1 eine ist, die aus den folgenden Verbindungen ausgewählt ist:

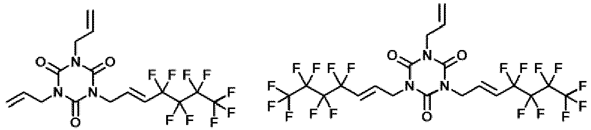

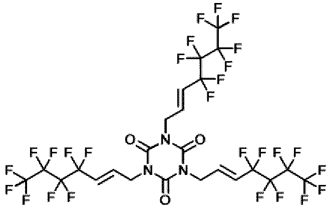

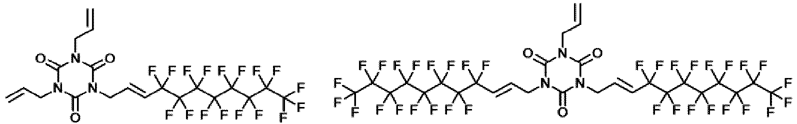

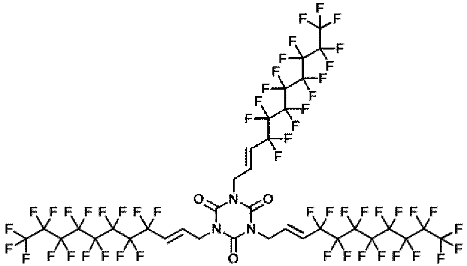

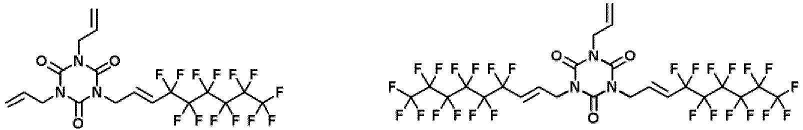

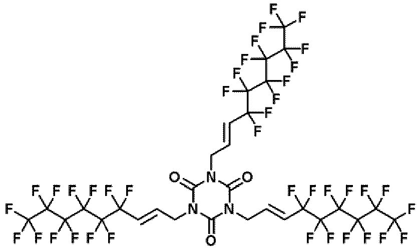

4. Verfahren zur Herstellung einer Verbindung der folgenden chemischen Formel 1, wobei das Verfahren umfasst:
(s1) Einbringen von Triallylisocyanurat, CₙF₂ₙ₊₁X4, einer ersten Base und eines ersten Lösungsmittels in einen Behälter und Rühren der resultierenden Mischung unter Stickstoffgas;
(s2) Zugeben eines Radikalinitiators dazu und Rühren der resultierenden Mischung; und
(s3) Zugeben eines zweiten Lösungsmittels und einer zweiten Base dazu, 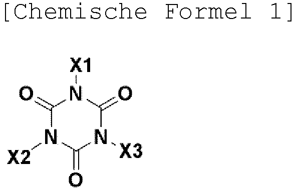
wobei in der chemischen Formel 1
mindestens eines von X1 bis X3 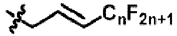 ist und die anderen eine Allylgruppe sind,
n eine ganze Zahl von 4 bis 12 ist, 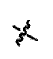 eine Einheit ist, die an die chemische Formel 1 gebunden ist, und
X4 eine Halogengruppe ist.

5. Mischung, umfassend zwei oder mehr der Verbindungen nach einem der Ansprüche 1 bis 3.

6. Oligomer, umfassend ein Monomer, das von der Verbindung nach einem der Ansprüche 1 bis 3 abgeleitet ist.

7. Polymer, umfassend ein Monomer, das von der Verbindung nach einem der Ansprüche 1 bis 3 abgeleitet ist.

8. Polymer nach Anspruch 7, wobei das Polymer ferner ein zusätzliches Monomer umfasst.

## Revendications

1. Composé de formule chimique 1 suivante: 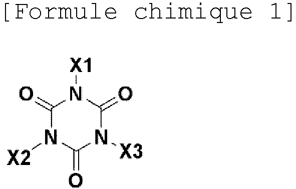 dans la formule chimique 1,
au moins l'un des symboles X1 à X3 représente 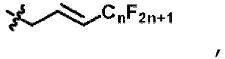
et les autres représentent un groupe allyle,
n est un nombre entier compris entre 4 et 12, et 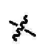 est un fragment lié à la formule chimique 1.

2. Composé de la revendication 1,
dans lequel le 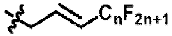 est l'une quelconque des structures suivantes:

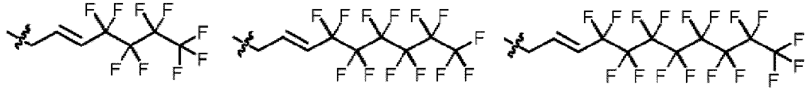

3. Composé de la revendication 1, dans lequel la formule chimique 1 est l'une quelconque des structures suivantes:

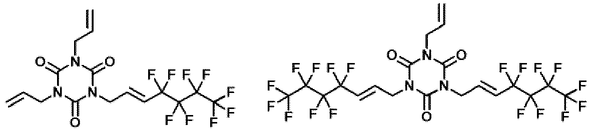

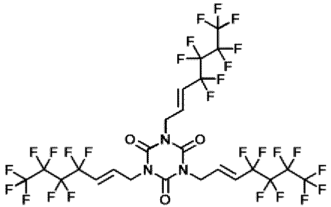

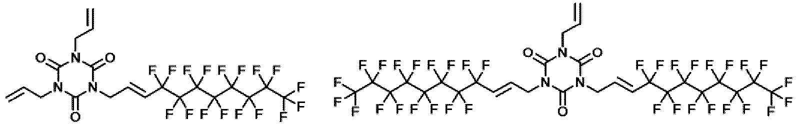

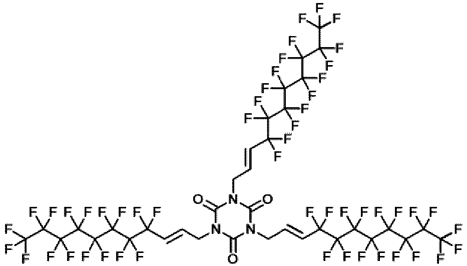

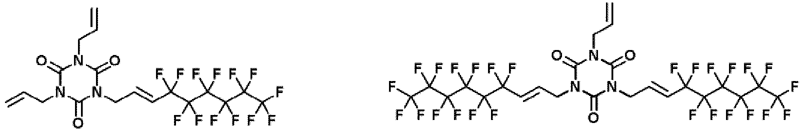

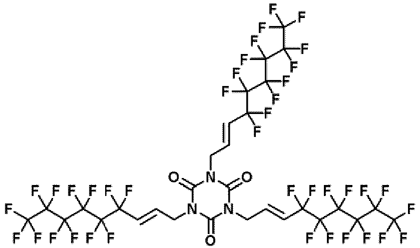

4. Méthode de préparation d'un composé de formule chimique 1 suivante, la méthode comprenant:
(s1) mettre de l'isocyanurate de triallyle, CₙF₂ₙ₊₁X4, une première base et un premier solvant dans un récipient et agiter le mélange obtenu sous azote gazeux;
(s2) ajouter un initiateur radicalaire et agiter le mélange obtenu; et
(s3) ajouter un deuxième solvant et une deuxième base, 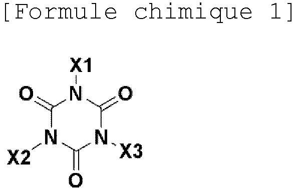
dans la formule chimique 1,
au moins l'un des X1 à X3 est 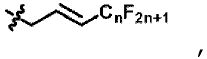
et les autres représentent un groupe allyle,
n est un nombre entier compris entre 4 et 12, 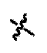 est un fragment lié à la formule chimique 1, et
X4 est un groupe halogène.

5. Mélange comprenant deux ou plusieurs des composés de l'une quelconque des revendications 1 à 3.

6. Oligomère comprenant un monomère dérivé du composé de l'une quelconque des revendications 1 à 3.

7. Polymère comprenant un monomère dérivé du composé de l'une quelconque des revendications 1 à 3.

8. Polymère de la revendication 7, dans lequel le polymère comprend en outre un monomère supplémentaire.
